# FASCICULE DE BREVET EUROPEEN

(11) **EP 2 670 858 B1**
(45) Date de publication et mention de la délivrance du brevet: **05.11.2014**
(21) Numéro de dépôt: 12707829.3
(22) Date de dépôt: 31.01.2012
(51) Int. Cl.: C12Q 1/04

(54) **MILIEU DE CULTURE DE MICROORGANISMES COMPRENANT L'ACIDE PARA-AMINOBENZOIQUE COMME AGENT SELECTIF**
KULTURMEDIUM FÜR MIKROORGANISMEN MIT PARA-AMINOBENZOESÄURE ALS SELEKTIONSMITTEL
CULTURE MEDIUM FOR MICROORGANISMS INCLUDING PARA-AMINOBENZOIC ACID AS A SELECTIVE AGENT

(30) Priorité: 01.02.2011 FR 1150768
(43) Date de publication de la demande: 11.12.2013
(73) Titulaire: Biomérieux, 69280 Marcy L'etoile (FR)
(72) Inventeur: ROCHE, Jean-Marc, F-73350 Feissons Sur Salins (FR)
(86) Numéro de dépôt international: PCT/FR2012/050207
(87) Numéro de publication internationale: WO 2012/104544

(56) Documents cités:
- WO-A1-02/22785
- WO-A1-02/24725
- WO-A1-96/40980
- WO-A1-98/04674
- WO-A2-01/66790
- US-A- 5 786 167
- US-A- 6 037 140
- R.M.E. RICHARDS ET AL: "Activity of p-aminobenzoic acid compared with other organic acids against selected bacteria", JOURNAL OF APPLIED MICROBIOLOGY, vol. 78, no. 3, 1 mars 1995 (1995-03-01), pages 209-215, XP055006907, ISSN: 1364-5072, DOI: 10.1111/j.1365-2672.1995.tb05018.x

## Description

Le domaine de l'invention est celui de l'analyse de microorganismes cibles dans un échantillon complexe.

Plus particulièrement, la présente invention concerne la détection de microorganismes tels les bactéries du genre *Salmonella,* au moyen d'un milieu de culture contenant de l'acide para-aminobenzoïque comme agent sélectif.

La qualité et la sécurité des produits mis sur le marché font partie des soucis majeurs de l'industrie agro-alimentaire, au niveau mondial. Dans les matières premières et les produits finis, il doit être possible de détecter, identifier et quantifier la flore microbienne et de garantir ainsi la valeur des produits, de leur fabrication jusqu'à leur consommation. Il est important de distinguer, d'une part les indicateurs-qualité du produit, reflet d'un taux de contamination global par une flore d'altération à laquelle appartiennent les entérobactéries comme *Escherichia coli,* les staphylocoques à coagulase positive, les *Pseudomonas,* les *Bacillus,* les levures, les moisissures, etc... et d'autre part la détection des pathogènes tels les bactéries du genre *Salmonella* ou *Listeria monocytogenes.* A ce titre, l'analyse microbiologique est primordiale en terme de santé publique et en terme économique pour cette industrie.

La mise au point de tests de détection de microorganismes cibles dans un échantillon répond à des contraintes fortes et nécessite de trouver un compromis entre la sensibilité, définie comme le pouvoir de mettre en évidence l'espèce recherchée, lorsque celle-ci est présente en faible quantité dans un échantillon à tester, et la sélectivité, définie comme le pouvoir de détecter l'espèce recherchée dans l'échantillon contenant également d'autres espèces. La combinaison des composants assurant la sensibilité, avec ceux assurant la sélectivité, permet d'obtenir un test spécifique.

Le critère de sensibilité impose fréquemment le recours à une composition permettant de cultiver les microorganismes cibles, afin de les rendre détectables. La spécificité peut ainsi être apportée par l'incorporation dans le milieu de culture d'une molécule-marqueur impliquée dans le métabolisme des microorganismes cibles ; cette interaction produisant un signal mesurable.

D'autre part, selon que l'on souhaite détecter et dénombrer un genre spécifique ou la totalité des germes viables, on pourra utiliser un milieu sélectif ou non.

Dans le cas de la recherche de microorganismes cibles, en particulier de bactéries, l'utilisation de milieux de culture chromogènes est de plus en plus importante. L'intérêt de tels milieux est de contenir des substrats enzymatiques spécifiquement clivés ou digérés par les bactéries cibles, de sorte qu'un produit de réaction coloré est généré, restant généralement localisé dans les colonies desdites bactéries. Ceci permet de différencier les dites bactéries cibles des autres genres bactériens éventuellement présents dans l'échantillon, en particulier, dans le cas d'échantillon agro-alimentaires. De tels milieux de culture permettent également l'identification d'une espèce bactérienne contenue dans un échantillon clinique, cette espèce étant généralement à l'origine d'une infection.

Dans le cas de la recherche de microorganismes cibles pathogènes dans des échantillons agroalimentaires, il apparaît généralement que le microorganisme cible est présent dans la population mixte de microorganismes et en plus faible quantité que les microorganismes non-cibles. En plus de la simple différenciation du microorganisme cible par rapport aux microorganismes non-cibles, il existe également la nécessité d'inhiber sélectivement la croissance desdits microorganismes non-cibles, afin de pouvoir détecter le microorganisme cible, à défaut de quoi, le développement desdits microorganismes non-cibles tendrait à masquer le microorganisme cible, quand bien même les colonies de ce dernier se retrouvent colorées, et ainsi conduire à des résultats faussement négatifs.

En particulier, il est pertinent de pouvoir différencier les bactéries du genre *Salmonella* d'autres types bactériens et notamment des bactéries gram négatives, telles que les bactéries de l'espèce *Escherichia coli (E. coli),* qui sont parmi les bactéries commensales présentes dans les échantillons.

A cette fin, le document WO-A-96/30543 décrit un milieu de culture pour la détection de *Salmonella* comprenant un substrat chromogène pour les espèces d'entérobactéries β-galactosidase positives telles qu'E. *coli* et un mélange de sucres métabolisés par les *Salmonella,* générant un composé acide et un indicateur de pH mettant en évidence l'acidification du milieu. Par ailleurs, des sels biliaires sont présents dans le milieu pour inhiber sélectivement les espèces gram positives.

Un méthode alternative permettant de détecter de façon spécifique les bactéries du genre *Salmonella* et de les différencier des bactéries gram négatives, a été décrit dans les documents EP-B-1 334 175 et EP-A-1 325 024. Cette méthode consiste à utiliser dans une milieu de culture, une agent sélectif se présentant sous la forme d'un substrat dit « suicide ». Un tel substrat est en fait constitué d'une partie dite « transporteur » et d'une partie dite « toxique », ces parties étant liées entre elles par une liaison destinée à être coupée par les bactéries *E. coli* et non par *Salmonella*, entraînant la libération et la concentration de la partie toxique à l'intérieur de *E. coli* et inhibant ainsi sa croissance, au profit de celle de *Salmonella*. Le substrat suicide de prédilection est l'alafosfaline.

Un tel substrat présente comme principal inconvénient d'entrer en compétition avec les acides aminés présents dans le milieu de culture. Aussi, pour qu'il soit actif sur un nombre important d'espèces bactériennes, il est nécessaire de disposer d'un milieu culture relativement pauvre en acides aminés, ce qui ne favorise pas la croissance des bactéries du genre *Salmonella.* De plus, l'alafosfaline est un composé relativement couteux, ce qui grève de façon importante le prix du milieu.

La présente invention permet de pallier ces inconvénients en proposant une solution consistant dans un milieu de culture comportant à titre d'agent sélectif, l'acide para-amino benzoïque. Un milieu de culture est ici défini comme un milieu comprenant tous les éléments nécessaires à la survie et/ou à la croissance de microorganismes, à savoir des acides aminés ou hydrolysats de protéines, des minéraux, des vitamines et d'une manière générale, tous les nutriments et toutes les sources métaboliques connus par l'homme du métier pour permettre la pousse microbienne.

Les inventeurs ont en effet démontré, de façon tout à fait surprenante, que l'addition d'acide para-amino benzoïque à concentration relativement élevée, dans un milieu de culture adapté, permet d'inhiber ou de ralentir en particulier la croissance des bactéries gram négatives, telle que les entérobactéries, au profit des bactéries du genre *Salmonella*.

Plus particulièrement, l'invention concerne un milieu de culture pour microorganismes comprenant :
- au moins un substrat, naturel ou synthétique, de fermentation ou d'activité enzymatique, et
- au moins un agent sélectif, constitué par l'acide para-amino benzoïque, un de ses dérivés ou un de leurs sels, à une concentration comprise entre 0,05 et 1 g/L, préférentiellement entre 0,05 et 0,8g/L.

De façon préférentielle, le microorganisme cible est une bactérie du genre *Salmonella*.

Les microorganismes non-cibles sont pris dans le groupe de bactéries gram négatives comprenant notamment les entérobactéries.

Un dérivé de l'acide para-amino benzoïque peut être pris dans le groupe comprenant notamment l'acide 4-amino benzoïque, l'acide 4-(méthylamino) benzoïque, l'acide 4-amino-2-chloro benzoïque, l'acide 4-amino-3-nitro benzoïque, l'acide 4-aminosalicylique.

Un sel acceptable peut être par exemple un sel de potassium ou un sel de sodium. On peut ainsi utiliser comme composé le 4-aminobenzoate de potassium ou encore le 4-aminobenzoate de sodium.

Avantageusement, le milieu de culture selon l'invention comporte au moins un substrat enzymatique chromogène ou fluorogène.

Préférentiellement, le milieu de culture selon l'invention comporte en outre des sels biliaires. Il peut par exemple comprendre du désoxycholate de sodium.

De façon avantageuse, ledit milieu de culture peut comporter au moins un autre agent sélectif pris dans le groupe comprenant : Novobiocine, Vancomycine, Amphothericine, Cefsulodine.

Au sens de la présente invention, le substrat est choisi parmi tout substrat pouvant être hydrolysé en un produit qui permet la détection, directe ou indirecte, d'une activité enzymatique spécifique du microorganisme recherché. Dans le cas de la détection directe, le substrat comprend une première partie spécifique de l'activité enzymatique et un deuxième partie faisant office de marqueur, qui peut être chromogène ou fluorescente.

Le substrat peut être également un substrat métabolique, tel qu'une source de carbone ou d'azote, dont le produit de dégradation fait varier le pH, variation détectable grâce à un indicateur de pH. L'indicateur de pH est une substance chimique dont la couleur varie en fonction de modifications du pH associées à la croissance microbienne. On citera comme exemples d'indicateur de pH, le rouge neutre, le bleu d'aniline, le bleu de bromocresol. Dans un second mode de réalisation, l'indicateur de pH est un fluorophore (par exemple la 4-méthylumbelliferone, les dérivés de coumarine ou les dérivés de la résorufine).

Au sens de l'invention, on entend par agent sélectif, tout composé susceptible de promouvoir la croissance d'un microorganisme cible et/ou inhiber la croissance d'un ou des microorganismes non cibles.

Le milieu de culture, objet de l'invention, peut être sous forme de poudre, sous forme de gel ou sous forme liquide, prêtes à l'emploi, c'est-à-dire prêtes pour l'ensemencement en tube, flacon, ou sur boite de Pétri. Dans le cas d'un milieu gélifié, l'agar est l'agent gélifiant traditionnel en microbiologie pour la culture des microorganismes, mais il est possible d'utiliser un autre agent gélifiant comme par exemple de la gélatine ou de l'agarose. Enfin, le milieu peut être conditionné dans une bouteille, une cartouche ou une carte spécifiques d'un appareil automatisé de bactériologie. on citera à titre d'exemple la carte Vitek®, la carte Tempo® et la bouteille BacT/ALERT®, commercialisées par la demanderesse.

L'invention concerne également un procédé de détection d'un microorganisme cible, dans une population mixte de cellules cibles et non cibles, comprenant les étapes consistant à :
- mettre en contact un échantillon dans lequel on souhaite mettre en évidence lesdits microorganismes cibles avec un milieu de culture selon l'invention ,
- incuber l'ensemble, à une température favorisant la multiplication desdits microorganismes,
- observer les microorganismes sur ledit milieu de culture.

La température favorisant la croissance des microorganismes est comprise entre 20 et 44°C et l'échantillon est maintenu à cette température pendant une durée suffisante pour permettre la détection des microorganismes, soit une durée comprise entre 2 et 96 heures. S'agissant de l'atmosphère d'incubation, elle est préférentiellement aérobie, mais elle peut également être anaérobie, microaérobie ou sous CO₂. L'identification peut être mise en oeuvre à l'oeil nu par visualisation d'un changement de coloration, ne diffusant pas dans le milieu de culture, donc concentrée au niveau des colonies. Dans le cas de la révélation de la fluorescence, on utilise les dispositifs de lecture de la fluorescence connus de l'homme du métier.

Le milieu de culture et la méthode de détection, objets de l'invention, sont utilisés pour des échantillons d'origine alimentaire, environnementale ou clinique. L'échantillon est défini comme une petite partie ou petite quantité isolée d'une entité pour l'analyse.

Parmi les échantillons d'origine alimentaire, on peut citer de façon non exhaustive un échantillon de produits lactés (yaourts, fromages...), de viande, de poisson, d'oeufs, de fruits, de légumes, d'eau, de boisson (lait, jus de fruits, soda, etc). Un échantillon alimentaire peut enfin être issu d'une alimentation destinée aux animaux, telle que notamment que des farines animales.

On mentionnera aussi les échantillons liés à l'environnement tels les prélèvements de surface, d'eau, d'air.

Les échantillons d'origine clinique peuvent correspondre à des prélèvements de sang total, de sérum, de plasma, d'urine, de fécès, de liquide céphalo-rachidien, des prélèvements de nez, de gorge, de peau, de plaie, d'organe, de tissu ou de cellules isolées etc.

L'invention concerne enfin l'utilisation *in vitro* de l'acide para-aminobenzoïque, un de ses dérivés ou un de leurs sels, à titre d'agent sélectif. De façon avantageuse, il est utilisé à une concentration comprise entre 0,05 et 1 g/L, préférentiellement entre 0,05 et 0,8g/L

Les exemples suivants sont donnés à titre illustratif et n'ont aucun caractère limitatif. Ils permettent de mieux comprendre l'invention.

### EXEMPLES

### Exemple 1 - Milieu de culture pour la détection de bactéries du genre Salmonella comprenant 3 substrats chromogènes, une combinaison d'antibiotiques (Novobiocine, Cefsulodine, vancomycine, amphotéricine) et différentes concentrations en acide para-aminobenzoïque (PABA)

### 1. Préparation des milieux testés

Les milieux testés sont les suivants:
- **Milieu Témoin :** milieu chromID® Salmonella (ref. 43621) commercialisé par la demanderesse contenant Novobiocine, Vancomycine, Amphothericine et Cefsulodine et les trois substrats chromogènes
- **Milieu 1 :** milieu Témoin, avec ajout de PABA 0,1 g/L,
- **Milieu 2 :** milieu Témoin, avec ajout de PABA 0,3 g/L,
- **Milieu 3 :** milieu Témoin, sans antibiotique et avec ajout de PABA 0,5 g/L,

Ces milieux sont des milieux en boite de Pétri 90 millimètres.

### 2. Ensemencement et lecture des milieux

Des souches de différentes espèces ou sérovars de *Salmonella (S. enteritidis, S. thyphimurium, S.* cubana, *S*. gallinarum, *S*. *paratyphi* par exemple) et d'autres genres microbiens tels que les bactéries gram négatives (*E. coli, Enterobacter, Citrobacter, Proteus, Hafnia),* toutes issues de la collection de la Demanderesse, ont été mises en suspension dans de l'eau physiologique, puis ensemencées sur les milieux, selon la technique des 4 quadrants. Les souches ont été incubées à 37°C pendant 24 heures. Les colonies formées ont été examinées visuellement après 24 heures d'incubation.

### 3. Résultats :

Les résultats obtenus sont présentés dans le tableau 1 ci-dessous.

**Tableau 1**

| **Milieu** | ***Salmonella* détectées / *Salmonella* testées** | **Autres souches Gram - différentes de *Salmonella* avec croissance / Autres souches Gram - différentes de *Salmonella* testées** |
|---|---|---|
| **Témoin** | 9/9 | 8/10 |
| **1** | 9/9 | 6/10 |
| **2** | 9/9 | 7/10 |
| **3** | 9/9 | 1/5 |

Ces résultats montrent une bonne sensibilité de détection des souches de *Salmonella* par tous les milieux de culture. En revanche, seuls les milieux 1 à 3 permettent de diminuer la croissance des autres bactéries à Gram négatives.

### Exemple 2 - Milieu de culture pour la détection de bactéries du genre Salmonella comprenant 3 substrats chromogenes, une combinaison d'éléments selectifs (Novobiocine, Cefsulodine, vancomycine, amphotericine, sels biliaires, désoxycholate de sodium) et de l'acide para-aminobenzoïque (PABA)

### 1. Préparation du milieu selon l'invention

Les milieux testés dans les expériences étaient les suivants:
- **Milieu Témoin** : milieu chromID® Salmonella (ref. 43621) contenant Novobiocine, Vancomycine, Amphothéricine, Cefsulodine, sels biliaires 1,5 g/L et 3 substrats chromogènes
- **Milieu 1** : milieu Témoin, avec ajout de PABA 0,1 g/L, sels biliaires augmentés à 3 g/L
- **Milieu 2** : milieu Témoin, avec ajout de PABA 0,1 g/L, sels biliaires augmentés à 3 g/L et désoxycholate de sodium à 5 g/L

### 2. Ensemencement et lecture des milieux

Des souches appartenant à différentes espèces ou sérovars de *Salmonella (S. enteritidis, S. thyphimurium, S.* cubana, *S.* gallinarum, *S. paratyphi, S. arizonae* par exemple) et d'autres genres microbiens, tels que les bactéries gram négatives (*Serratia, Enterobacter, Citrobacter, Proteus, Hafnia*)toutes issues de la collection de la Demanderesse, ont été mises en suspension dans de l'eau physiologique, puis ensemencées sur les milieux, selon la technique des quatre quadrants. Les boîtes ont été incubées à 37°C pendant 24 heures. Les colonies formées ont été examinées visuellement après 24 heures d'incubation.

### 3. Résultats :

Les résultats obtenus sont présentés dans le tableau 2, ci-après.

**Tableau 2**

| **Milieu** | ***Salmonella* détectées** / ***Salmonella* testées** | **Autres genres bactériens différents de *Salmonella* avec croissance / autres genres bactériens testés** |
|---|---|---|
| **T** | 9/10 | 5/6 |
| **1** | 9/10 | 3/6 |
| **2** | 10/10 | 1/6 |

Ces résultats montrent une excellente sensibilité de tous les milieux pour la recherche des souches de *Salmonella,* détection de la totalité des 10 souches de *Salmonella* pour la formule 2. En revanche, seuls les milieux 1 et 2 permettent de diminuer la croissance des autres genres bactériens différents de *Salmonella.* La meilleure sélectivité est obtenue en associant le PABA avec des sels biliaires tels que le désoxycholate de sodium.

## Revendications

1. Milieu de culture pour la détection d'au moins un microorganisme cible comprenant :
• au moins un substrat, naturel ou synthétique, de fermentation ou d'activité enzymatique, et
• au moins un agent sélectif, permettant l'inhibition des microorganismes non cibles, constitué par l'acide para-amino benzoïque, un de ses dérivés ou un de leurs sels, à une concentration comprise entre 0,05 et 1 g/L, préférentiellement entre 0,05 et 0,8g/L.

2. Milieu de culture selon la revendication 1, dans lequel le microorganisme cible est une bactérie du genre *Salmonella*.

3. Milieu de culture selon la revendication 1 ou 2, dans lequel les microorganismes non-cibles sont pris dans le groupe comprenant notamment les entérobactéries.

4. Milieu de culture selon l'une des revendications précédentes, dans lequel le dérivé de l'acide para-amino benzoïque est pris dans le groupe comprenant : l'acide 4-amino benzoïque, l'acide 4-(méthylamino) benzoïque, l'acide 4-amino-2-chloro benzoïque, l'acide 4-amino-3-nitro benzoïque, l'acide 4-aminosalicylique.

5. Milieu de culture selon l'une des revendication précédentes, comportant en outre au moins un substrat enzymatique chromogène ou fluorogène.

6. Milieu de culture selon l'une des revendications précédentes, comprenant en outre des sels biliaires.

7. Milieu de culture selon l'une des revendications précédentes, comprenant comme sel biliaire, du désoxycholate de sodium.

8. Milieu de culture selon l'une des revendications précédentes, au moins un autre agent sélectif pris dans le groupe comprenant : Novobiocine, Vancomycine, Amphothericine, Cefsulodine.

9. Procédé de détection d'un microorganisme cible, dans une population mixte de cellules cibles et non cibles, comprenant les étapes consistant à :
• mettre en contact un échantillon dans lequel on souhaite mettre en évidence lesdits microorganismes cibles avec un milieu de culture selon l'une des revendication 1 à 8,
• incuber l'ensemble, à une température favorisant la multiplication desdits microorganismes
• observer les microorganismes sur ledit milieu de culture.

10. Utilisation *in vitro* de l'acide para-amino benzoïque, un de ses dérivés ou un de leurs sels, dans un milieu de culture comme agent sélectif.

11. Utilisation selon la revendication précédente, dans laquelle l'acide para-aminobenzoïque, un de ses dérivés ou un de leurs sels, est présent dans le milieu de culture à une concentration comprise entre 0,05 et 1g/L, préférentiellement entre 0,05 et 0,8g/L.

## Patentansprüche

1. Kulturmedium für den Nachweis von mindestens einem Zielmikroorganismus umfassend:
• mindestens ein natürliches oder synthetisches Fermentations- oder Enzymaktivitätssubstrat und
• mindestens ein Selektionsmittel, das die Hemmung von Nichtzielmikroorganismen gestattet, bestehend aus para-Aminobenzoesäure, einem ihrer Derivate öder einem ihrer Salze, in einer Konzentration zwischen 0,05 und 1 g/l, vorzugweise zwischen 0,05 und 0,8 g/l.

2. Kulturmedium nach Anspruch 1, wobei es sich bei dem Zielmikroorganismus um ein Bakterium der Gattung *Salmonella* handelt.

3. Kulturmedium nach Anspruch 1 oder 2, wobei die Nichtzielmikroorganismen aus der Gruppe umfassend insbesondere die Enterobakterien ausgewählt sind.

4. Kulturmedium nach einem der vorhergehenden Ansprüche, wobei das para-Aminobenzoesäurederivat aus der Gruppe umfassend 4-Aminobenzoesäure, 4-(Methylamino)benzoesäure, 4-Amino-2-chlorbenzoesäure, 4-Amino-3-nitrobenzoesäure und 4-Aminosalicylsäure ausgewählt ist.

5. Kulturmedium nach einem der vorhergehenden Ansprüche, das weiterhin mindestens ein chromogenes oder fluorogenes Enzymsubstrat umfasst.

6. Kulturmedium nach einem der vorhergehenden Ansprüche, das weiterhin Gallensalze umfasst.

7. Kulturmedium nach einem der vorhergehenden Ansprüche, das als Gallensalz Natriumdesoxycholat umfasst.

8. Kulturmedium nach einem der vorhergehenden Ansprüche, das mindestens ein weiteres Selektionsmittel, ausgewählt aus der Gruppe umfassend Novobiocin, Vancomycin, Amphothericin und Cefsulodin, umfasst.

9. Verfahren zum Nachweisen eines Zielmikroorganismus in einer Mischpopulation von Ziel- und Nichtzielzellen, das die Schritte bestehend aus Folgendem umfasst:
• Inkontaktbringen einer Probe, in der man die Zielmikroorganismen nachzuweisen wünscht, mit einem Kulturmedium nach einem der Ansprüche 1 bis 8,
• Inkubieren des Ganzen bei einer Temperatur, die die Vermehrung dieser Mikroorganismen begünstigt,
• Beobachten der Mikroorganismen auf dem Kulturmedium.

10. In-vitro-Verwendung von para-Aminobenzoesäure, einem ihrer Derivate oder einem ihrer Salze als Selektionsmittel in einem Kulturmedium.

11. Verwendung nach dem vorhergehenden Anspruch, wobei die para-Aminobenzoesäure, eines ihrer Derivate oder eines ihrer Salze in dem Kulturmedium in
einer Konzentration zwischen 0,05. und 1 g/l, vorzugsweise zwischen 0,05 und 0,8 g/l, vorliegt.

## Claims

1. Culture medium for detecting at least one target microorganism, comprising:
• at least one natural or synthetic substrate for fermentation or for enzymatic activity, and
• at least one selective agent, making it possible to inhibit the non-target microorganisms, consisting of para-aminobenzoic acid, one of its derivatives or one of their salts, at a concentration of between 0.05 and 1 g/l, preferentially between 0.05 and 0.8 g/l.

2. Culture medium according to Claim 1, in which the target microorganism is a bacterium of the *Salmonella* genus.

3. Culture medium according to Claim 1 or 2, in which the non-target microorganisms are taken from the group comprising in particular enterobacteria.

4. Culture medium according to one of the preceding claims, in which the para-aminobenzoic acid derivative is taken from the group comprising: 4-aminobenzoic acid, 4-(methylamino)benzoic acid, 4-amino-2-chlorobenzoic acid, 4-amino-3-nitrobenzoic acid and 4-aminosalicylic acid.

5. Culture medium according to one of the preceding claims, also comprising at least one chromogenic or fluorogenic enzymatic substrate.

6. Culture medium according to one of the preceding claims, also comprising bile salts.

7. Culture medium according to one of the preceding claims, comprising sodium deoxycholate as bile salt.

8. Culture medium according to one of the preceding claims, comprising at least one other selective agent taken from the group comprising: novobiocin, vancomycin, amphotericin and cefsulodin.

9. Method for detecting a target microorganism, in a mixed population of target and non-target cells, comprising the steps consisting in:
• bringing a sample in which it is desired to demonstrate said target microorganisms into contact with a culture medium according to one of Claims 1 to 8,
• incubating the whole at a temperature which promotes the multiplication of said microorganisms,
• observing the microorganisms on said culture medium.

10. *In vitro* use of para-aminobenzoic acid, one of its derivatives or one of their salts, in a culture
medium as selective agent.

11. Use according to the preceding claim, in which the para-aminobenzoic acid, one of its derivatives or one of their salts, is present in the culture medium at a concentration of between 0.05 and 1 g/l, preferentially between 0.05 and 0.8 g/l.
